# EUROPÄISCHE PATENTSCHRIFT

(11) **EP 0 498 255 B1**
(45) Veröffentlichungstag und Bekanntmachung des Hinweises auf die Patenterteilung: **11.10.1995**
(21) Anmeldenummer: 92101290.2
(22) Anmeldetag: 27.01.1992
(51) Int. Cl.: A61B 6/00, G03B 42/02

(54) **Strahlenuntersuchungsgerät für Brustuntersuchungen**
Radiographic apparatus for mammography
Dispositif radiologique de mammographie

(30) Priorität: 05.02.1991 SE 9100362
(43) Veröffentlichungstag der Anmeldung: 12.08.1992
(73) Patentinhaber: Siemens-Elema AB, 171 95 Solna 1 (SE); SIEMENS AKTIENGESELLSCHAFT, 80333 München (DE)
(72) Erfinder: Warden, Hans, Ing., S-194 51 Upplands Väsby (SE)

(56) Entgegenhaltungen:
- EP-A- 0 127 073
- WO-A-89/11248

## Beschreibung

Die Erfindung betrifft ein Strahlenuntersuchungsgerät insbesondere für Brustuntersuchungen mit einer Halterung, die einen Strahlenkopf enthält, wobei die Halterung über eine horizontale Achse um ein in einem Stativ vertikal verschiebbar angeordnetes Lager drehbar ist, das durch eine Gewichtsausgleichsvorrichtung beeinflußt wird.

Ein Strahlenuntersuchungsgerät dieser Art ist durch den Siemens-Prospekt "Mammomat 2" vom November 1987 bekannt. Bei diesem Röntgengerät für Brustuntersuchungen ist die Halterung für die Röntgenröhre um die erwähnte horizontale Achse, die im Schwerpunkt der Halterung angeordnet ist, drehbar. Die Drehung der Halterung um die Achse erfolgt mit Hilfe einer konventionellen Drehvorrichtung. Die vertikale Verschiebung der Halterung erfolgt mit Hilfe eines Motors und einer Zahnstange, die von der Drehvorrichtung der Halterung getrennt angeordnet sind.

Bei einer Routineuntersuchung der Brust des Patienten erfolgt eine erste vertikale Aufnahme und eine zweite Aufnahme mit einem Winkel von 45° bis 60° ausgehend davon. Bei der Drehung der Halterung von der ersten zu der zweiten Aufnahmestellung muß die Halterung in der Höhe neu eingestellt werden. Außerdem muß der Patient einen Schritt zur Seite machen, weil die Drehung der Halterung außerhalb der Zentrumachse des Aufnahmeobjekts erfolgt.

Dieses Problem wird in der in WO 89/112 48 beschriebenen Vorrichtung dadurch gelöst, daß die Achse für die Halterung außerhalb des Schwerpunktes der Halterung selbst angeordnet ist. Ferner offenbart WO 89/11248 eine Gasfederung zum Gewichtsausgleich der Halterung während ihrer Drehung.

Der Erfindung liegt die Aufgabe zugrunde, ein Strahlenuntersuchungsgerät der eingangs genannten Art zu schaffen, bei dem die Bewegungen der Halterung mit Hilfe von verhältnismäßig einfachen Mitteln erfolgen können und bei dem die Halterung so gedreht werden kann, daß diese von einer Aufnahme zu einer weiteren Aufnahme mit einem beliebigen Winkel ihre vertikale Lage behält gleichzeitig damit, daß der Patient auf der gleichen Stelle stehen bleiben kann.

Diese Aufgabe ist erfindungsgemäß dadurch gelöst, daß die Achse für die Halterung außerhalb des Schwerpunktes der Halterung angeordnet und mit einem herausragenden ersten Teil verbunden ist, das parallel zur Achse und mit Abstand zu dessen Zentrumlinie angeordet ist, wobei das herausragende erste Teil in ein zweites Teil, das in vertikaler Richtung bewegbar und mit der Gewichtsausgleichsvorrichtung verbunden ist, eingreift, wobei der Abstand zwischen dem ersten Teil und der Zentrumlinie der Achse so gewählt ist, daß beim Drehen der Achse das erste Teil auf einem Kreisbogen verschoben und das zweite Teil in einer vertikalen Richtung derart verschoben wird, daß durch die Gewichtsausgleichsvorrichtung eine Kraft erzeugt wird, die das Drehmoment der Halterung ausbalanciert. Durch diesen Aufbau wird eine gemeinsame Gewichtsausgleichsvorrichtung für die vertikale Verschiebung der Halterung und deren Drehung um ihre horizontale Achse erzielt, wobei die Achse nunmehr etwa mit der Mitte des Untersuchungsobjekts zusammenfällt. Auf diese Weise wird erreicht, daß weder die Halterung noch der Patient bei einer Änderung des Aufnahmewinkels umpositioniert werden muß.

In einer Weiterbildung der Erfindung wird vorgeschlagen, daß das zweite Teil in vertikaler Richtung gesteuert wird. Auf diese Weise wird eine stabile vertikale Bewegung erhalten.

In einer vorteilhaften Ausbildung der Erfindung wird vorgeschlagen, daß das zweite Teil mit einem horizontalen Schlitz versehen ist, in dem das erste Teil verschiebbar ist. Hierdurch ist eine sichere Verbindung zwischen den beiden Teilen gegeben. Dies wird auch erreicht, in dem das zweite Teil mit einem horizontalen Aufsatz versehen ist, auf dem das erste Teil läuft.

In einer konstruktiv einfachen Ausgestaltung der Erfindung wird vorgeschlagen, daß das erste Teil mit einer Laufrolle versehen ist. Dadurch wird erreicht, daß das erste Teil im Schlitz oder auf dem Aufsatz leicht läuft.

Eine günstige Ausgestaltung der Erfindung ergibt sich, wenn das zweite Teil mit einer Rolle versehen ist, über die ein Seil verläuft, wobei das eine Ende des Seils mit dem Lager für die Achse und das zweite Ende des Seils mit der Gewichtsausgleichsvorrichtung verbunden ist. Hierdurch wird eine Übersetzung der Gleichgewichtskraft derart erhalten, daß nur ein verhältnismäßig kurzer Abstand zwischen der Zentrumlinie der Achse und dem freien Ende eines kurbelförmigen Teils, das an dem einen Ende der Achse befestigt ist und das die Laufrolle trägt, notwendig ist. Auf diese Weise wird der Raumbedarf vermindert.

Weitere Vorteile und Einzelheiten ergeben sich aus den Unteransprüchen.

Die Erfindung ist nachfolgend anhand eines Ausführungsbeispieles in Verbindung mit den Zeichungen näher erläutert. Es zeigen:
- FIG 1: eine Seitenansicht eines Strahlenuntersuchungsgerät nach der Erfindung und
- FIG 2: eine Draufsicht des strahlenuntersuchungsgerät gemäß FIG 1.

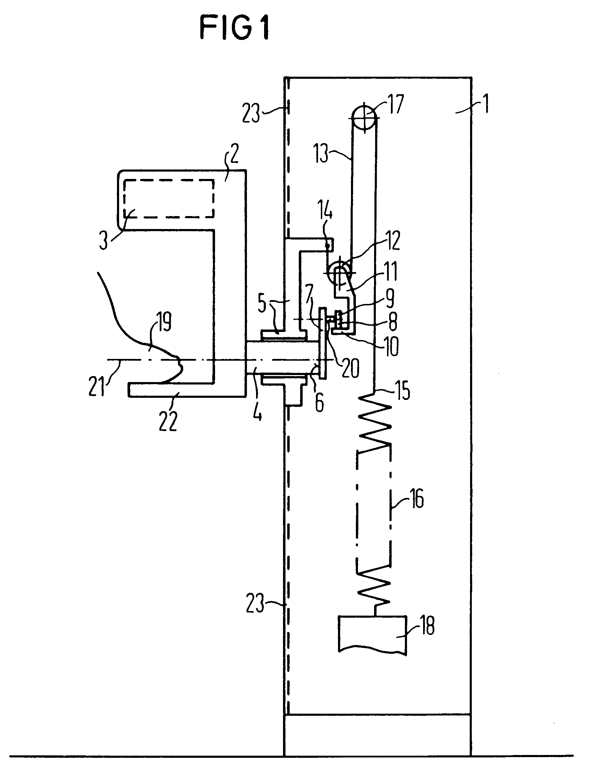

In der FIG 1 ist ein Röntgengerät für Brustuntersuchungen schematisch dargestellt mit einem Stativ 1 und einer Halterung 2, die eine Röntgenröhre 3 trägt. Die Halterung 2 ist über eine horizontale Achse 4 mit einem in dem Stativ 1 angeordnetes Lager 5, das in vertikaler Richtung verschiebbar ist, drehbar verbunden. Die freie Endseite 6 der Achse 4 ist mit einem kurbelwellenförmigen Teil 7 versehen, dessen freies Ende 20 von der Achse 4 wegweist, wobei das freie Ende 20 zur Achse 4 parallel und mit Abstand zur Zentrumlinie 21 der Achse 4 angeordnet ist. An dem freien Ende 2 des kurbelförmigen Teils 7 ist eine Laufrolle 8 angebracht, deren Lauffläche 9 gegen einem Aufsatz 10 anliegt, der zu einem Momentbalancierwagen 11 gehört. Der Wagen 11, der in vertikaler Richtung verschiebbar gesteuert ist, ist mit einer Rolle 12 versehen, über die ein Seil 13 verläuft, wobei das eine Ende 14 des Seiles 13 mit dem Lager 5 und das andere Ende 15 des Seiles 13 mit einer Gewichtsausgleichsvorrichtung 16,17 verbunden ist. Die Gewichtsausgleichsvorrichtung 16,17 besteht in diesem Ausführungsbeispiel aus einer Gleichgewichtstrommel 17 und einer Zugfeder 16, die über eine Befestigungsstelle 18 am Stativ 1 befestigt ist. Durch die Gleichgewichtstrommel 17 ist die Gleichgewichtskraft unabhängig von der Lage der Zugfeder 16 konstant. Die Rolle 12 bildet einen Flaschenzug, durch den die Gleichgewichtskraft übersetzt wird. Der Momentbalancierwagen 11 kann anstatt des Aufsatzes 10 einen Schlitz aufweisen, in dem das freie Ende des kurbelförmigen Teils 7 oder die Laufrolle 8 verschiebbar sind.

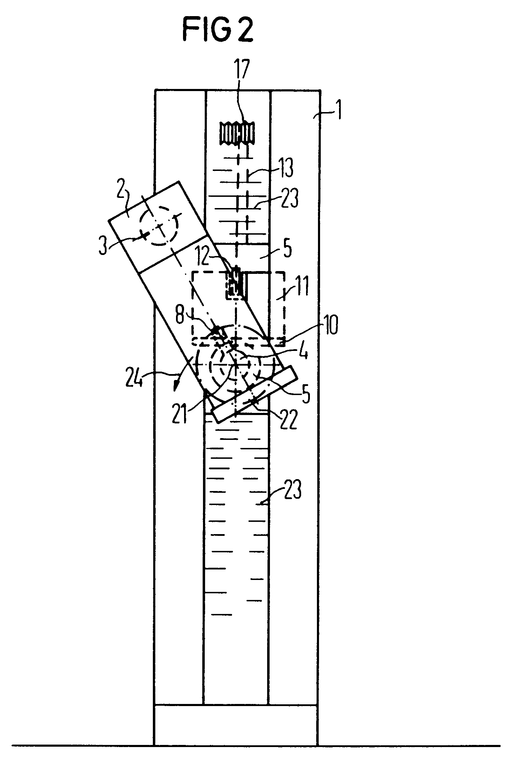

Aus der FIG 1 und 2 geht hervor, daß jalousieähnliche Abdeckungen 23 am Lager 5 befestigt sind, die die vertikale Öffnung am Stativ 1, in der das Lager 5 läuft, schließen. Die Abdeckungen 23 verhindern, daß Staub in das Stativ 1 eindringt.

Bei einer Routineuntersuchung einer Brust 19 eines Patienten wird die Halterung 2 und ein Objekttisch 22, der an der Halterung 2 befestigt ist, in eine vertikale Lage, die an die Körperlänge des Patienten angepaßt ist, verschoben. Dabei wird die Halterung 2 mit der Achse 4 und das Lager 5 nach oben oder nach unten geschoben, wobei der Wagen 11 durch das Seil 13 dieser Bewegung folgt. Durch die Gewichtsausgleichsvorrichtung 16,17 ist die Halterung 2 in jeder Lage ausbalanciert. Nun wird die Brust 19 auf dem Objekttisch 22 plaziert, um, wie in FIG 1 gezeigt, eine senkrechte Aufnahme zumachen. Dann wird die Halterung 2 um einen Winkel von 45° bis 60° für eine weitere Brustaufnahme gedreht. Eine solche Lage der Halterung 2 ist in der FIG 2 gezeigt. Wenn die Halterung 2 um ihre Achse 4 gedreht wird, wird auch das kurbelförmige Teil 7 gedreht, wodurch die Laufrolle 8 den Aufsatz 10 und damit auch den Wagen 11 nach unten drückt. Durch dieselbe Gewichtsausgleichsvorrichtung 16,17, die die Halterung 2 gewichtsmäßig ausbalanciert, wird nun eine Kraft erzeugt, die das Drehmoment der Haltung 2 ausgleicht, wodurch die Halterung 2 in der beschriebenen Lage bleibt. Bei einer genaueren Nachuntersuchung wählt der Arzt zu die bereits beschriebenen häufig weitere Aufnahmewinkel. Die Halterung 2 kann z.B. in die Richtung des Pfeiles 24 gedreht werden, wodurch der Wagen 11 durch die Laufrolle 8, die gegen den Aufsatz 10 drückt, nach unten verschoben wird. So kann die Halterung um ihre Achse um 360° gedreht werden und ist in jeder beliebigen Winkellage ausbalanciert, so daß ein beliebiger Aufnahmewinkel einstellbar ist.

Aufgrund des Aufbaus des Strahlenuntersuchungsgerät nach der Erfindung kann die Achse 4 für die Halterung 2 außerhalb des Schwerpunktes der Halterung 2 angebracht werden. Durch Verlegen der Achse 4, d.h. des Drehpunktes der Halterung 2 ins Zentrum des Untersuchungsobjekts 19 ist es nicht notwendig, die Halterung 2 beim Wechseln des Aufnahmewinkels in der Höhe einzustellen. Außerdem kann der Patient beim Wechseln der Aufnahmewinkel stehen bleiben, was sehr zeitsparend ist.

## Patentansprüche

1. Strahlenuntersuchungsgerät insbesondere für Brustuntersuchungen mit einer Halterung (2), die einen Strahlenkopf (3) enthält, wobei die Halterung (2) über eine horizontale Achse (4) um ein in einem Stativ vertikal verschiebbar angeordnetes Lager (5) drehbar ist, das durch eine Gewichtsausgleichsvorrichtung (16,17) beeinflußt wird, **dadurch gekennzeichnet,** daß die Achse (4) für die Halterung (2) außerhalb des Schwerpunktes der Halterung (2) angeordnet und mit einem herausragenden ersten Teil (20) verbunden ist, das parallel zur Achse (4) und mit Abstand zu dessen Zentrumlinie (21) angeordnet ist, wobei das herausragende erste Teil (20) in ein zweites Teil (11), das in vertikaler Richtung bewegbar und mit der Gewichtsausgleichsvorrichtung (16,17) verbunden ist, eingreift, wobei der Abstand zwischen dem ersten Teil (20) und der Zentrumlinie (21) der Achse (4) so gewählt ist, daß beim Drehen der Achse (4) das erste Teil (20) auf einem Kreisbogen verschoben und das zweite Teil (11) in einer vertikalen Richtung derart verschoben wird, daß durch die Gewichtsausgleichsvorrichtung (16,17) eine Kraft erzeugt wird, die das Drehmoment der Halterung (2) ausbalanciert.

2. Strahlenuntersuchungsgerät nach Anspruch 1, **dadurch gekennzeichnet,** daß das zweite Teil (11) in vertikaler Richtung gesteuert wird.

3. Strahlenuntersuchungsgerät nach Anspruch 1 oder 2, **dadurch gekennzeichnet,** daß das zweite Teil (11) mit einem horizontalen Schlitz versehen ist, in dem das erste Teil (20) verschiebbar ist.

4. Strahlenuntersuchungsgerät nach Anspruch 1 oder 2, **dadurch gekennzeichnet,** daß das zweite Teil (11) mit einem horizontalen Aufsatz (10) versehen ist, auf dem das erste Teil (20) läuft.

5. Strahlenuntersuchungsgerät nach Anspruch 3 oder 4, **dadurch gekennzeichnet,** daß das erste Teil (20) mit einer Laufrolle (8) versehen ist.

6. Strahlenuntersuchungsgerät nach einem der Ansprüche 1 bis 5, **dadurch gekennzeichnet,** daß das zweite Teil (11) mit einer Rolle (12) versehen ist, über die ein Seil (13) verläuft, wobei das eine Ende (14) des Seils (13) mit dem Lager (5) für die Achse (4) und das zweite Ende (15) des Seils (13) mit der Gewichtsausgleichsvorrichtung (16,17) verbunden ist.

7. Strahlenuntersuchungsgerät nach Anspruch 6, **dadurch gekennzeichnet,** daß die Rolle (12) als Flaschenzug dient.

8. Strahlenuntersuchungsgerät nach einem der Ansprüche 1 bis 7, **dadurch gekennzeichnet,** daß das Drehzentrum (21) der Halterung (2) etwa mit der Mitte des Untersuchungsobjekts (19) zusammenfällt.

9. Strahlenuntersuchungsgerät nach einem der Ansprüche 1 bis 8, **dadurch gekennzeichnet,** daß die Halterung (2) einen Objekttisch (22) trägt.

## Claims

1. Radiographic apparatus, especially for mammography, having a mounting (2) which contains a radiation head (3), the mounting (2) being rotatable by means of a horizontal axle (4) about a bearing (5) which is arranged vertically displaceably in a stand and is influenced by a weight compensating device (16, 17), characterized in that the axle (4) for the mounting (2) is arranged outside the centre of gravity of the mounting (2) and is connected to a projecting first part (20) which is arranged parallel to the axle (4) and at a distance from its centre line (21), the projecting first part (20) engaging in a second part (11), which is movable in the vertical direction and is connected to the weight compensating device (16, 17), the distance between the first part (20) and the centre line (21) of the axle (4) being selected such that, during rotation of the axle (4), the first part (20) is displaced on a circular arc and the second part (11) is displaced in a vertical direction in such a way that a force which balances the torque of the mounting (2) is generated by the weight compensating device (16, 17).

2. Radiographic apparatus according to Claim 1, characterized in that the second part (11) is controlled in the vertical direction.

3. Radiographic apparatus according to Claim 1 or 2, characterized in that the second part (11) is provided with a horizontal slot in which the first part (20) is displaceable.

4. Radiographic apparatus according to Claim 1 or 2, characterized in that the second part (11) is provided with a horizontal attachment (10) on which the first part (20) runs.

5. Radiographic apparatus according to Claim 3 or 4, characterized in that the first part (20) is provided with a running roller (8).

6. Radiographic apparatus according to one of Claims 1 to 5, characterized in that the second part (11) is provided with a roller (12) over which a cable (13) runs, the one end (14) of the cable (13) being connected to the bearing (5) for the axle (4) and the second end (15) of the cable (13) being connected to the weight compensating device (16, 17).

7. Radiographic apparatus according to Claim 6, characterized in that the roller (12) serves as a block and tackle.

8. Radiographic apparatus according to one of Claims 1 to 7, characterized in that the centre of rotation (21) of the mounting (2) approximately coincides with the centre of the object to be examined (19).

9. Radiographic apparatus according to one of Claims 1 to 8, characterized in that the mounting (2) carries an object table (22).

## Revendications

1. Appareil d'examen radiologique, notamment pour des examens mammographiques, comportant un support (2), qui contient une tête d'irradiation (3), le support (2) pouvant tourner autour d'un axe horizontal (4), dans un palier (5) disposé de manière à être déplaçable verticalement dans un statif et qui est influencé par un dispositif de contrepoids (16,17), caractérisé par le fait que l'axe (4) pour le support (2) est disposé en dehors du centre de gravité du support (2) et est relié à une première partie saillante (20), qui est disposée parallèlement à l'axe (4) et à distance de sa ligne centrale (21), la première partie saillante (20) s'engageant dans une seconde partie (11), qui est déplaçable verticalement et qui est reliée au dispositif de contrepoids (16,17), la distance entre la première partie (20) et la ligne centrale (21) de l'axe (4) étant choisie de telle sorte que, lors de la rotation de l'axe (4), la première partie (20) est déplacée sur un arc de cercle et la seconde partie (11) est déplacée dans une direction verticale de telle sorte que le dispositif de contrepoids (16,17) produit une force qui équilibre le couple du support (2).

2. Appareil d'examen radiologique suivant la revendication 1, caractérisé par le fait que la seconde partie (11) est commandée dans une direction verticale.

3. Appareil d'examen radiologique suivant la revendication 1 ou 2, caractérisé par le fait que la seconde partie (11) est pourvue d'une fente horizontale, dans laquelle la première partie (20) est déplaçable.

4. Appareil d'examen radiologique suivant la revendication 1 ou 2, caractérisé par le fait que la seconde partie (11) est pourvue d'une partie saillante horizontale (10), sur laquelle circule la première partie (20)

5. Appareil d'examen radiologique suivant la revendication 3 ou 4, caractérisé par le fait que la première partie (20) comporte un galet de roulement (8).

6. Appareil d'examen radiologique suivant l'une des revendications 1 à 5, caractérisé par le fait que la seconde partie (11) est pourvue d'un galet (12), sur lequel circule un câble (13), une extrémité (14) du câble (13) étant reliée au papier (5) pour l'axe (4), tandis que la seconde extrémité (15) du câble (13) est reliée au dispositif de contrepoids (16,17).

7. Appareil d'examen radiologique suivant la revendication 6, caractérisé par le fait que le galet (12) est utilisé en tant que mouffle.

8. Appareil d'examen radiologique suivant l'une des revendications 1 à 7, caractérisé par le fait que le centre de rotation (21) du support (2) coïncide approximativement avec le centre de l'objet à examiner (19).

9. Appareil d'examen radiologique suivant l'une des revendications 1 à 8, caractérisé par le fait que le support (2) porte une table porte-objet (22).
